(19) Europäisches Patentamt
European Patent Office
Office européen des brevets

(11) EP 2 772 491 A1

(12) EUROPEAN PATENT APPLICATION
published in accordance with Art. 153(4) EPC

(43) Date of publication:
**03.09.2014 Bulletin 2014/36**

(21) Application number: **12841899.3**

(22) Date of filing: **19.10.2012**

(51) Int Cl.:
***C07D 403/12*** *(2006.01)*
***A61K 31/506*** *(2006.01)*
***A61P 5/28*** *(2006.01)*
***A61P 13/08*** *(2006.01)*
***A61P 35/00*** *(2006.01)*

(86) International application number:
**PCT/JP2012/077091**

(87) International publication number:
**WO 2013/058361 (25.04.2013 Gazette 2013/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.10.2011 JP 2011231273**

(71) Applicant: **Astellas Pharma Inc.**
**Chuo-ku**
**Tokyo 103-8411 (JP)**

(72) Inventors:
- **NAKAMURA, Takashi**
  **Tokyo 103-8411 (JP)**
- **OKAMOTO, Takumi**
  **Tokyo 103-8411 (JP)**
- **HIRAKURA, Yutaka**
  **Tokyo 103-8411 (JP)**
- **YAMAZAKI, Kouji**
  **Tokyo 103-8411 (JP)**
- **KAKEFUDA, Akio**
  **Tokyo 103-8411 (JP)**
- **YAMASHITA, Hiroyuki**
  **Tokyo 103-8411 (JP)**

(74) Representative: **Bates, Philip Ian**
**Reddie & Grose LLP**
**16 Theobalds Road**
**London WC1X 8PL (GB)**

# (54) CRYSTALS OF ANDROGEN RECEPTOR ANTAGONIST COMPOUND

(57) [Problem] A crystal of (2R,5S)-4-(3-chloro-4-cyanophenyl)-N-(2-cyclopropylpyrimidin-5-yl)-2,5-dimethylpiperazine-1-carboxamide with excellent activity as an androgen receptor (AR) antagonistic compound, the salts thereof, or the solvate thereof, which is suitable for industrialization as a bulk drug of pharmaceutical products; a pharmaceutical composition comprising the crystal as an active ingredient; and the pharmaceutical composition which is the AR antagonistic drug are provided.

[Solution] A01-type crystal of monohydrate, A01-type crystal of hydrochloride, A01-type crystal of hydrobromide, and A01-type crystal of methanesulfonate of (2R, 5S)-4-(3-chloro-4-cyanophenyl)-N-(2-cyclopropylpyrimidin-5-yl)-2,5-dimethylpiperazine-1-carboxamide, which have certain crystal forms, are provided.

Fig. 1

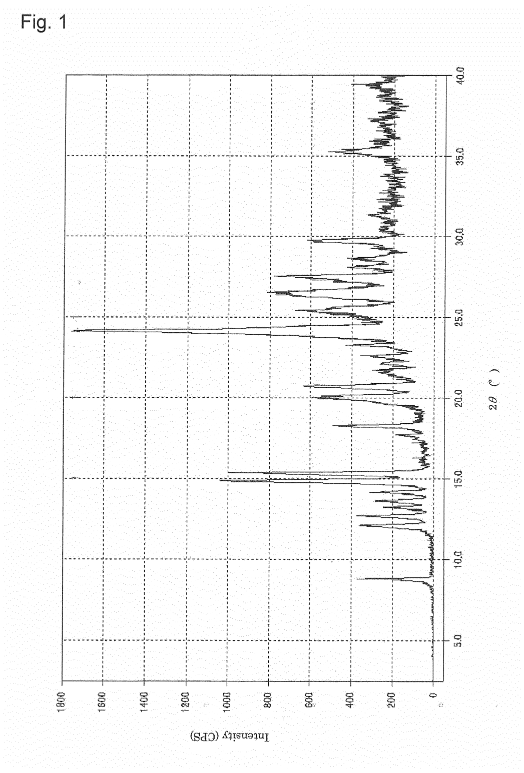


EP 2 772 491 A1

Printed by Jouve, 75001 PARIS (FR)

## Description

Technical Field

The present invention relates to a crystal of (2R,5S)-4-(3-chloro-4-cyanophenyl)-N-(2-cyclopropylpyrimidin-5-yl)-2,5-dimethylpiperazine-1-carboxamide (hereinafter, also referred to as "Compound A"), salts thereof, or solvates thereof, which is suitable for industrialization as a bulk drug of pharmaceutical products; a pharmaceutical composition comprising the crystal as an active ingredient; and an androgen receptor (AR) antagonistic drug comprising the pharmaceutical composition.

Background Art

The present inventors previously found that Compound A represented by the following formula (I) (a compound of Examples 3 to 9 in Patent Document 1) has an AR antagonistic action, excellent oral activity in a rat, and a prostate-shrinking effect, and is useful as an agent for treating various diseases on which androgen acts as an exacerbation factor, particularly for treating prostate cancer and benign prostatic hyperplasia (Patent Document 1).
Here, in the industrialization of pharmaceutical products, a bulk drug which has a certain degree of quality and storage stability and can be stably supplied in a large quantity is required for the production and the improvement of the pharmaceutical products. Therefore, in regard to the industrialization of Compound A as an AR antagonistic drug, it is necessary to acquire a suitable bulk drug and it is necessary to acquire a crystal suitable for the industrialization in obtaining the bulk drug.
As a crystal suitable for the industrialization, a crystal in which a uniform crystal having a certain degree of quality can be obtained with excellent reproducibility and which can be stably supplied as a crystal of a bulk drug used for production of pharmaceuticals and is suitable for mass synthesis in the industrial production is desired. It is further desired that the obtained crystal have low hygroscopicity and excellent storage stability from excellent photostability.
In general, many compounds serving as an active ingredient of pharmaceuticals can be isolated as free bodies or as a solid by forming salts with a base or an acid. In addition, when a crystal is produced, a different crystal is produced from the obtained solid according to various crystallization conditions in some cases.
However, to be used as a crystal suitable for the industrialization as a bulk drug of pharmaceuticals, a crystal is desired to have a series of the above-described properties, however an examination for many salts and the crystallization thereof are required because the properties are not easily acquired.
In regard to Compound A, a synthesis method is disclosed in Patent Document 1 below, but the compound, the salts thereof, the crystal of the solvate thereof, or a production method thereof is not disclosed or suggested.
As described above, the crystal suitable for the industrialization of Compound A, the salts thereof, or the solvate thereof is strongly demanded.

[Chem. 1]

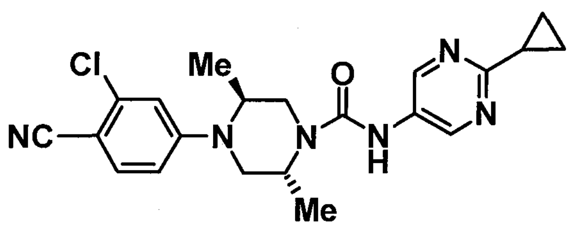

(I)

Example 22 of Patent Document 2 discloses a monohydrate crystal of Compound A, which was added at the filing date of the application of Patent Document 2. The filing date of Patent Document 2 is the same date as the priority date of the present application.
[Patent Document 1] Pamphlet of International Publication WO 2004/007471
[Patent Document 2] Pamphlet of International Publication WO 2012/053630

Disclosure of Invention

Problems to Be Solved by the Invention

An object of the present invention is to provide a crystal of Compound A with excellent activity as an AR antagonistic drug, which is suitable for industrialization as a bulk drug of pharmaceutical products, a pharmaceutical composition comprising the crystal as an active ingredient, and the pharmaceutical composition which is the AR antagonistic drug.

Means for Solving the Problems

The present inventors examined a plurality of salts and crystallization conditions to acquire a crystal suitable for industrialization of Compound A as a bulk drug of pharmaceutical products.
Specifically, crystallization examination is performed on Compound A, hydrochloride of Compound A, hydrobromide of Compound A, methanesulfonate of Compound A, toluenesulfonate of Compound A, sulfate of Compound A, sodium salts of Compound A, potassium salts of Compound A, calcium salts of Compound A, various solvents, and crystallization conditions (that is, Slry: slurry method, Evp: evaporation method, Cool: cooling method, Ppt: precipitation method by addition of poor solvent).
As a result, crystalline solids in which two kinds are present in hydrochloride, six kinds in hydrobromide, one kind in methanesulfonate, one kind in toluenesulfonate, and one kind in sulfate, that have different spectra from each other are respectively acquired.
Several crystalline solids are obtained as a result of the examination, but most of the obtained crystalline solids are not crystals suitable for the industrialization for the reasons such as certain crystal forms are not obtained, the thermodynamic stability of each crystal form is not stable, or the like.
Through trial and error of further crystallization examination, the present inventors successfully acquired the crystal which has a certain crystal form and is suitable for the industrialization in a monohydrate of Compound A obtained under specific crystallization conditions and pharmaceutically acceptable several salts, thereby completing the present invention.
In other words, the present invention relates to (1) to (23) described below.

(1) (2R,5S)-4-(3-chloro-4-cyanophenyl)-N-(2-cyclopropylpyrimidin-5-yl)-2,5-dimethylpiperazine-1-carboxamide monohydrate.
(2) A crystal of (2R,5S)-4-(3-chloro-4-cyanophenyl)-N-(2-cyclopropylpyrimidin-5-yl)-2,5-dimethylpiperazine-1-carboxamide monohydrate.
(3) The crystal according to (2), which has an endothermic peak having an extrapolated onset temperature which falls within the range of 75°C to 110°C in a differential scanning calorimetry analysis (DSC analysis).
(4) The crystal according to (2), which has peaks at approximately 14.9°, 15.4°, 20.1°, 20.7°, 24.1°, 25.4°, 26.5°, 27.5°, and 29.8° of diffraction angles 2θ (°) in powder X-ray diffraction (tube: Cu).
(5) The crystal according to (2), which has an endothermic peak having an extrapolated onset temperature which falls within the range of 75°C to 110°C in a differential scanning calorimetry analysis (DSC analysis), and peaks at approximately 14.9°, 15.4°, 20.1°, 20.7°, 24.1°, 25.4°, 26.5°, 27.5°, and 29.8° of diffraction angles 20 (°) in powder X-ray diffraction (tube: Cu).
(6) A crystal of (2R,5S)-4-(3-chloro-4-cyanophenyl)-N-(2-cyclopropylpyrimidin-5-yl)-2,5-dimethylpiperazine-1-carboxamide hydrochloride.
(7) The crystal according to (6), which has an endothermic peak having an extrapolated onset temperature which falls within the range of 160°C to 169°C in the differential scanning calorimetry analysis (DSC analysis).
(8) The crystal according to (6), which has peaks at approximately 13.2°, 16.2°, 19.5°, 24.7°, 25.1°, 26.1°, and 27.7° of diffraction angles 2θ (°) in powder X-ray diffraction (tube: Cu).
(9) The crystal according to (6), which has an endothermic peak having an extrapolated onset temperature which falls within the range of 160°C to 169°C in the differential scanning calorimetry analysis (DSC analysis), and peaks at approximately 13.2°, 16.2°, 19.5°, 24.7°, 25.1°, 26.1°, and 27.7° of diffraction angles 2θ (°) in powder X-ray diffraction (tube: Cu).
(10) A crystal of (2R,5S)-4-(3-chloro-4-cyanophenyl)-N-(2-cyclopropylpyrimidin-5-yl)-2,5-dimethylpiperazine-1-carboxamide hydrobromide.
(11) The crystal according to (10), which has an endothermic peak having an extrapolated onset temperature which falls within the range of 185°C to 195°C in the differential scanning calorimetry analysis (DSC analysis).
(12) The crystal according to (10), which has peaks at approximately 16.0°, 19.3°, 21.6°, 23.0°, 24.0°, 24.4°, 25.0°, 26.2°, 27.2°, and 30.4° of diffraction angles 20 (°) in powder X-ray diffraction (tube: Cu).

(13) The crystal according to (10), which has an endothermic peak having an extrapolated onset temperature which falls within the range of 185°C to 195°C in the differential scanning calorimetry analysis (DSC analysis), and peaks at approximately 16.0°, 19.3°, 21.6°, 23.0°, 24.0°, 24.4°, 25.0°, 26.2°, 27.2°, and 30.4° of diffraction angles 2θ (°) in powder X-ray diffraction (tube: Cu).
(14) A crystal of (2R,5S)-4-(3-chloro-4-cyanophenyl)-N-(2-cyclopropylpyrimidin-5-yl)-2,5-dimethylpiperazine-1-carboxamide methanesulfonate.
(15) The crystal according to (14), which has an endothermic peak having an extrapolated onset temperature which falls within the range of 185°C to 195°C in the differential scanning calorimetry analysis (DSC analysis).
(16) The crystal according to (14), which has peaks at approximately 16.6°, 18.3°, 20.3°, 23.9°, 24.4°, 27.1 °, and 28.1° of diffraction angles 2θ (°) in powder X-ray diffraction (tube: Cu).
(17) The crystal according to (14), which has an endothermic peak having an extrapolated onset temperature which falls within the range of 185°C to 195°C in the differential scanning calorimetry analysis (DSC analysis), and peaks at approximately 16.6°, 18.3°, 20.3°, 23.9°, 24.4°, 27.1°, and 28.1° of diffraction angles 2θ (°) in powder X-ray diffraction (tube: Cu).
(18) A pharmaceutical composition, which comprises the crystal according to any one of (2) to (17) or (2R,5S)-4-(3-chloro-4-cyanophenyl)-N-(2-cyclopropylpyrimidin-5-yl)-2,5-dimethylpiperazine-1-carboxamide monohydrate according to (1) as an active ingredient.
(19) The pharmaceutical composition according to (18), which is an androgen receptor antagonistic drug.
(20) The crystal according to any one of (2) to (17) or (2R,5S)-4-(3-chloro-4-cyanophenyl)-N-(2-cyclopropylpyrimidin-5-yl)-2,5-dimethylpiperazine-1-carboxamide monohydrate according to (1) for treating prostate cancer.
(21) Use of the crystal according to any one of (2) to (17) or (2R,5S)-4-(3-chloro-4-cyanophenyl)-N-(2-cyclopropylpyrimidin-5-yl)-2,5-dimethylpiperazine-1-carboxamide monohydrate according to (1) for the manufacture of a pharmaceutical composition for treating prostate cancer.
(22) Use of the crystal according to any one of (2) to (17) or (2R,5S)-4-(3-chloro-4-cyanophenyl)-N-(2-cyclopropylpyrimidin-5-yl)-2,5-dimethylpiperazine-1-carboxamide monohydrate according to (1) for treating prostate cancer.
(23) A method of treating prostate cancer, which comprises administering to a subject an effective amount of the crystal according to any one of (2) to (17) or (2R,5S)-4-(3-chloro-4-cyanophenyl)-N-(2-cyclopropylpyrimidin-5-yl)-2,5-dimethylpiperazine-1-carboxamide monohydrate according to (1).

In addition, the "subject" refers to a human or other animals in need of the prevention or the treatment, and in a certain embodiment, a human in need of the prevention or the treatment.

Effects of the Invention

According to the present invention, there are provided a crystal of Compound A with excellent activity as an AR antagonistic drug, the salts thereof, or the solvate thereof, which is suitable for industrialization as a bulk drug of pharmaceutical products; a pharmaceutical composition comprising the crystal as an active ingredient; and the pharmaceutical composition which is the AR antagonistic drug.

Brief Description of Drawings

[Fig. 1] Fig. 1 is a powder X-ray diffraction pattern of "A01-type crystal of monohydrate" of Compound A (the crystal of the present invention).
[Fig. 2] Fig. 2 is a powder X-ray diffraction pattern of "A01-type crystal of hydrochloride" of Compound A (the crystal of the present invention).
[Fig. 3] Fig. 3 is a powder X-ray diffraction pattern of "A01-type crystal of hydrobromide" of Compound A (the crystal of the present invention).
[Fig. 4] Fig. 4 is a powder X-ray diffraction pattern of "A01-type crystal of methanesulfonate" of Compound A (the crystal of the present invention).
[Fig. 5] Fig. 5 is a powder X-ray diffraction pattern of Compound A (the compound of Examples 3 to 9 in Patent Document 1, amorphous).
[Fig. 6] Fig. 6 is a differential scanning calorimetry analysis curve (DSC analysis curve) of "A01-type crystal of monohydrate" of Compound A (the crystal of the present invention).
[Fig. 7] Fig. 7 is a differential scanning calorimetry analysis curve (DSC analysis curve) of "A01-type crystal of monohydrate" of Compound A (the crystal of the present invention), in a lot which is different from the lot of Fig. 6.
[Fig. 8] Fig. 8 is a differential scanning calorimetry analysis curve (DSC analysis curve) of "A01-type crystal of

hydrochloride" of Compound A (the crystal of the present invention).

[Fig. 9] Fig. 9 is a differential scanning calorimetry analysis curve (DSC analysis curve) of "A01-type crystal of hydrobromide" of Compound A (the crystal of the present invention).

[Fig. 10] Fig. 10 is a differential scanning calorimetry analysis curve (DSC analysis curve) of "A01-type crystal of methanesulfonate" of Compound A (the crystal of the present invention).

[Fig. 11] Fig. 11 is a differential scanning calorimetry analysis curve (DSC analysis curve) of Compound A (a compound of Examples of 3 to 9 in Patent Document 1, amorphous).

Embodiments for Carrying Out the Invention

"A01-type crystal of monohydrate", "A01-type crystal of hydrochloride", "A01-type crystal of hydrobromide", and "A01-type crystal of methanesulfonate" of Compound A of the present invention can be obtained with excellent reproducibility as a uniform crystal having a certain degree of quality under specific controllable conditions. In addition, these crystals can be stably supplied and suitable for the industrialization of Compound A.

The "A01-type crystal of monohydrate" of Compound A indicates an endothermic peak having any extrapolated starting temperature which falls within the range of 75°C to 110°C and any endothermic peak top temperature which falls within the range of 90°C to 115°C in the differential scanning calorimetry analysis (DSC analysis). These numerical ranges are derived from a plurality of lots in which the A01-type crystal of monohydrate of Compound A is produced. The reason that the numerical range is relatively wide is the influence of volatilization of water molecules solvated in Compound A at the time of heating during the DSC analysis, and it is considered that melting crystals is an equilibrium phenomenon which is controlled and measured by a certain degree of temperature specific to crystals, while the volatilization of water molecules has an aspect which indicates kinetic dependency. That is, the temperature when water molecules are separated or volatilized varies depending on the particle size of a crystal sample and the way that the crystal sample is put over a measurement sample pan, and the like. Further, unevenness of the endothermic peaks in the DSC analysis curve are observed in not only lots of crystals which are different from each other but also lots which are the same as each other, and the endothermic peaks vary in every measurement preparation (see Fig. 7).

Hereinafter, the present invention will be described in detail.

"An androgen receptor antagonistic drug (AR antagonistic drug)" means a drug used for treating a disease in which androgen acts as an exacerbation factor, for example, prostate cancer.

"Monohydrate" of Compound A means a hydrate in which one molecule of water is hydrated with respect to one molecule of Compound A.

The "A01-type crystal of monohydrate" of Compound A is a crystal of monohydrate of Compound A, in which one molecule of water is hydrated with respect to one molecule of Compound A, and which has peaks at approximately 14.9°, 15.4°, 20.1 °, 20.7°, 24.1°, 25.4°, 26.5°, 27.5°, and 29.8° of diffraction angles 2θ (°) in powder X-ray diffraction (tube: Cu). As another embodiment, the "A01-type crystal of monohydrate" of Compound A is a crystal of monohydrate of Compound A, in which one molecule of water is hydrated with respect to one molecule of Compound A, and which has an endothermic peak having an extrapolated onset temperature which falls within the range of 75°C to 110°C in a differential scanning calorimetry analysis (DSC analysis), and/or peaks at approximately 14.9°, 15.4°, 20.1°, 20.7°, 24.1°, 25.4°, 26.5°, 27.5°, and 29.8° of diffraction angles 20 (°) in powder X-ray diffraction (tube: Cu). As still another embodiment, the "A01-type crystal of monohydrate" of Compound A is a crystal of monohydrate of Compound A which is shown as the powder X-ray diffraction pattern of Fig. 1 and/or the DSC analysis curve of Fig. 6 or Fig. 7, or a diagram which can be identified with those.

Furthermore, there is no known crystal type other than monohydrate crystal of Compound A.

The "A01-type crystal of hydrochloride" of Compound A is a crystal of hydrochloride of Compound A, in which one molecule of Compound A and one molecule of hydrochloric acid form salts, and which has an endothermic peak having an extrapolated onset temperature which falls within the range of 160°C to 169°C in a differential scanning calorimetry analysis (DSC analysis), and/or peaks at approximately 13.2°, 16.2°, 19.5°, 24.7°, 25.1°, 26.1°, and 27.7° of diffraction angles 2θ (°) in powder X-ray diffraction (tube: Cu). As another embodiment, the "A01-type crystal of hydrochloride" of Compound A is a crystal of hydrochloride of Compound A which is shown as the powder X-ray diffraction pattern of Fig. 2 and/or the DSC analysis curve of Fig. 8, or a diagram which can be identified with those.

In addition, as the crystal of hydrochloride of Compound A, it is confirmed that an A02-type crystal of hydrochloride is present in addition to the A01-type crystal thereof.

The "A01-type crystal of hydrobromide" of Compound A is a crystal of hydrobromide of Compound A, in which one molecule of Compound A and one molecule of hydrobromic acid form salts, and which has an endothermic peak having an extrapolated onset temperature which falls within the range of 185°C to 195°C in a differential scanning calorimetry analysis (DSC analysis), and/or peaks at approximately 16.0°, 19.3°, 21.6°, 23.0°, 24.0°, 24.4°, 25.0°, 26.2°, 27.2°, and 30.4° of diffraction angles 2θ (°) in powder X-ray diffraction (tube: Cu). As another embodiment, the "A01-type crystal of hydrobromide" of Compound A is a crystal of hydrobromide of Compound A which is shown as the powder

X-ray diffraction pattern of Fig. 3 and/or the DSC analysis curve of Fig. 9, or a diagram which can be identified with those.

In addition, as the crystal of hydrobromide of Compound A, it is confirmed that A02-type crystal of hydrobromide and A03-type crystal of hydrobromide are present in addition to the A01-type crystal thereof.

The "A01-type crystal of methanesulfonate" of Compound A is a crystal of methanesulfonate of Compound A, in which one molecule of Compound A and one molecule of methanesulfonic acid form salts, and which has an endothermic peak having an extrapolated onset temperature which falls within the range of 185°C to 195°C in a differential scanning calorimetry analysis (DSC analysis), and/or peaks at approximately 16.6°, 18.3°, 20.3°, 23.9°, 24.4°, 27.1°, and 28.1° of diffraction angles 2θ (°) in powder X-ray diffraction (tube: Cu). As another embodiment, the "A01-type crystal of methanesulfonate" of Compound A is a crystal of methanesulfonate of Compound A which is shown as the powder X-ray diffraction pattern of Fig. 4 and/or the DSC analysis pattern of Fig. 10, or a diagram which can be identified with those.

In addition, as the methanesulfonate crystal of Compound A, it is confirmed that A02-type crystal of methanesulfonate is present in addition to the A01-type crystal thereof.

Compound A described in Examples 3 to 9 disclosed in Patent Document 1 is amorphous and is shown as the powder X-ray diffraction pattern of Fig. 5 and the DSC analysis curve of Fig. 11.

Further, with regard to the powder X-ray diffraction, crystal lattice distance or the entire patterns are important for confirming the identity of the crystal due to the properties of data, and the relative intensity can be slightly changed depending on the direction of crystal growth, the size of particles, and measurement conditions, so the intensity should not be interpreted strictly. In addition, the present invention relates to the pure monohydrate of Compound A, the "A01-type crystal of monohydrate", the "A01-type crystal of hydrochloride", the "A01-type crystal of hydrobromide", and the "A01-type crystal of methanesulfonate" of Compound A, but mixtures which can be substantially identified with monohydrate of the pure Compound A, the "A01-type crystal of monohydrate", the "A01-type crystal of hydrochloride", the "A01-type crystal of hydrobromide", and the "A01-type crystal of methanesulfonate" of Compound A are also included in the scope of the present invention.

In numerical values obtained from various spectra, some errors happen to occur due to the direction of the crystal growth, the size of particles, and measurement conditions. Accordingly, in consideration of those errors, the term "approximately" used for values of the diffraction angles 2θ in the powder X-ray diffraction of the present specification means that the angle value is approximately the defined value of the diffraction angle 2θ, and the angle value may be in the range of ± 1° of the value as a certain embodiment, and the angle value may be in the range of ± 0.5° of the value as another embodiment, and the angle value may be in the range of ± 0.2° of the value as still another embodiment. "5. Qualitative analysis (phase identification)" of "Chapter 2.58 Powder X-ray diffraction measurement method" in "Japanese Pharmacopoeia" describes that the "diffraction angles 2θ between the basic substances and the materials of the same crystal forms coincide with each other within 0.2°" particularly in measurement of organic crystals.

The "powder X-ray diffraction" and the "differential scanning calorimetry analysis (DSC analysis)" were respectively measured under the following conditions.

(Powder X-ray diffraction)

The powder X-ray diffraction of the crystals of the present invention was measured by using "MAC Science MXP18TAHF22" under the conditions of tube: Cu, tube current: 40 mA, tube voltage: 200 kV, sampling width: 0.020°, scanning speed: 4°/min, wavelength: 1.54056 Å, and measurement diffraction angle range (2θ): 2.5° to 40°.

(Differential scanning calorimetry analysis (DSC analysis))

The differential scanning calorimetry analysis of the crystals of the present invention was measured by using "TA Instrument TA 2910" under the conditions at a temperature of room temperature to 300°C (10°C/min), with $N_2$ (50 ml/min), and with an aluminum sample pan.

(Preparation method)

Compound A monohydrate can be produced by a conventional method using Compound A produced in conformity to Examples 3 to 9 of Patent Document 1 or by a method which can be generally used by those skilled in the art. For example, Compound A monohydrate can be produced by mixing Compound A with water or a mixed solvent prepared from water and an organic solvent mixed at a particular ratio, dissolving Compound A by heating as needed, and collecting the solid deposited thereafter. As the mixed solvent prepared from water and an organic solvent mixed at a particular ratio, for example, acetonitrile water, ethanol water, acetone water, or dioxane water can be used, and acetonitrile water can be used as another embodiment. The water content of the mixed solvent may be, for example, 30% to 80%, 40% to 80%, 50% to 70%, or 50%. As the acetonitrile water, for example, 20% to 80% of acetonitrile water, 30% to 60% of acetonitrile water, or 40% to 50% of acetonitrile water can be used, and 50% acetonitrile water can be

used as another embodiment.

As described above, as the crystal of Compound A monohydrate, only the A01 type crystal has been found and another crystal form has not been found. Therefore, the A01 type crystal of Compound A monohydrate is produced by the preparation method of the above-described Compound A monohydrate. In addition, the A01 type crystal of Compound A monohydrate can be produced by recrystallizing Compound A monohydrate produced by the preparation method of Compound A monohydrate using the mixed solvent formed of water and an organic solvent mixed at a particular ratio as described above.

The A01 type crystal of Compound A hydrochloride can be produced by recrystallizing Compound A hydrochloride produced by a conventional method using Compound A produced in conformity to Examples 3 to 9 of Patent Document 1 or by a method which can be generally used by those skilled in the art and using a mixed solvent of water and an appropriate solvent such as acetonitrile, ethanol, methanol, or an organic solvent of those. Compound A hydrochloride can be produced by applying hydrogen chloride in an appropriate solvent, and can be produced using acetonitrile as a solvent as a certain embodiment.

Further, the A01 type crystal of Compound A hydrochloride can be produced by applying hydrogen chloride to Compound A produced in conformity to Examples 3 to 9 of Patent Document 1 or by a method which can be generally used by those skilled in the art in an appropriate solvent such as acetonitrile, ethanol, methanol, or a mixed solvent of water and those organic solvent, followed by dissolution by heating as needed, and collection of the solid deposited thereafter.

Here, the water content of the mixed solvent of the organic solvent and water is, for example, 5% to 30% or 10% to 20%.

The A01 type crystal of Compound A hydrobromide can be produced by recrystallizing Compound A hydrobromide produced by a conventional method using Compound A produced in conformity to Examples 3 to 9 of Patent Document 1 or by a method which can be generally used by those skilled in the art and using an appropriate solvent such as acetonitrile, 2-propanol, or a mixed solvent of water and those organic solvent. Compound A hydrobromide can be produced by applying hydrobromic acid in an appropriate solvent, and can be produced using acetonitrile as a solvent as a certain embodiment.

Further, the A01 type crystal of Compound A hydrobromide can be produced by applying hydrobromic acid to Compound A produced in conformity to Examples 3 to 9 of Patent Document 1 or by a method which can be generally used by those skilled in the art in an appropriate solvent such as acetonitrile, 2-propanol, or a mixed solvent of water and those organic solvent, followed by dissolution by heating as needed, and collection of the solid deposited thereafter.

Here, the water content of the mixed solvent of the organic solvent and water is, for example, 5% to 30% or 10% to 20%.

The A01 type crystal of Compound A methanesulfonate can be produced by recrystallizing Compound A methanesulfonate produced by a conventional method using Compound A produced in conformity to Examples 3 to 9 of Patent Document 1 or by a method which can be generally used by those skilled in the art and using an appropriate solvent, for example, a mixed solvent of acetonitrile and methanol, acetonitrile, or acetonitrile water. Compound A methanesulfonate can be produced by applying methanesulfonic acid in an appropriate solvent, and can be produced using acetonitrile, tetrahydrofuran, or dioxane as a solvent as a certain embodiment.

Further, the A01 type crystal of Compound A methanesulfonate can be produced by applying methanesulfonic acid to Compound A produced in conformity to Examples 3 to 9 of Patent Document 1 or by a method which can be generally used by those skilled in the art in an appropriate solvent such as acetonitrile, tetrahydrofuran, dioxane, a mixed solvent of acetonitrile and methanol, or acetonitrile water, followed by dissolved by heating as needed, and collection of the solid deposited thereafter.

Here, the water content of the acetonitrile water is, for example, 5% to 30% or 10% to 20%. The ratio of the mixed solvent of acetonitrile to methanol may be, as one side thereof, 10% to 90%, 20% to 80%, 30% to 70%, or 40% to 80%.

The pharmaceutical composition of the present invention comprises the "A01-type crystal of monohydrate", the "A01-type crystal of hydrochloride", the "A01-type crystal of hydrobromide", or the "A01-type crystal of methanesulfonate" of Compound A, or any combination of one or more kinds thereof as an active ingredient, further comprises a pharmaceutically acceptable carrier, and is useful as an AR antagonistic drug. In addition, the pharmaceutical composition of the present invention is useful as a drug for treating prostate cancer.

Pharmaceutical compositions comprising one or more kinds of the crystal or monohydrate of the present invention as an active ingredient can be prepared using excipients that are usually used in the art, that is, excipients for pharmaceutical preparation, carriers for pharmaceutical preparation, and the like according to the methods usually used.

Administration can be accomplished either by oral administration via tablets, pills, capsules, granules, powders, solutions, and the like, or parenteral administration, such as injections such as intraarticular, intravenous, and intramuscular injections, suppositories, ophthalmic solutions, eye ointments, transdermal solutions, ointments, transdermal patches, transmucosal solutions, transmucosal patches, inhalers, and the like.

Solid compositions for oral administration are used in the form of tablets, powders, granules, or the like. In such solid compositions, one or more active ingredient(s) are mixed with at least one inactive excipient. In a conventional method, the composition may comprise inactive additives, such as lubricants, disintegrating agents, stabilizers, or solubilization assisting agents. If necessary, tablets or pills may be coated with sugar or s gastric- or enteric-soluble substances films.

Liquid compositions for oral administration comprises pharmaceutically acceptable emulsions, solutions, suspensions, syrups, elixirs, or the like, and also comprises generally used inert diluents, for example, purified water or ethanol (EtOH). In addition to the inert diluent, liquid compositions may also comprise auxiliary agents, such as solubilization assisting agents, moistening agents, and suspending agents, sweeteners, flavors, aromatics, or antiseptics.

Injections for parenteral administration include sterile aqueous or non-aqueous solutions, suspensions, or emulsions. Aqueous solvents include, for example, distilled water for injection or physiological saline. Examples of non-aqueous solvents include alcohols such as ethanol. Such compositions may further comprise tonicity agents, antiseptics, moistening agents, emulsifying agents, dispersing agents, stabilizers, or solubilization assisting agents. These are sterilized, for example, by filtration through bacteria retaining filter, blendings of bactericide, or irradiation. In addition, these can also be used by preparing sterile solid compositions, and dissolving or suspending in sterile water or sterile solvents for injection prior to its use.

Agents for external use includes ointments, plasters, creams, jellies, poultices, sprays, lotions, eye drops, eye ointments, and the like. The agents comprise generally used ointment bases, lotion bases, aqueous or non-aqueous solutions, suspensions, emulsions, and the like.

As transmucosal agents such as inhalers, transnasal agents, and the like, those in the form of a solid, liquid, or semi-solid state are used, and can be prepared in accordance with conventionally known methods. For example, known excipients, and furthermore pH adjusting agents, antiseptics, surfactants, lubricants, stabilizers, thickening agents, or the like may be appropriately added thereto. For their administration, appropriate devices for inhalation or blowing can be used. For example, a compound may be administered alone or as a powder of formulated mixture, or as a solution or suspension in combination with pharmaceutically acceptable carriers, using a known device or sprayer, such as a measured administration inhalation device, and the like. Dry powder inhalers or the like may be for single or multiple administration use, and dry powder or powder-containing capsules may be used. Alternatively, these may be pressurized aerosol spray which uses appropriate ejection agents, for example, a suitable gas such as chlorofluoroalkane, carbon dioxide, and the like.

For oral administration, daily dose is generally from about 0.001 to 100 mg/kg, preferably from 0.1 to 30 mg/kg, and more preferably from 0.1 to 10 mg/kg, per body weight, administered in one portion or in 2 to 4 separate portions. In the case of intravenous administration, daily dose is suitably administered from about 0.0001 to 10 mg/kg per body weight, once a day or two or more times a day. In addition, a transmucosal agent is administered at a dose from about 0.001 to 100 mg/kg per body weight, once a day or two or more times a day. Doses are appropriately determined according to the individual according to the symptoms, age, gender, and the like.

Although varying depending on administration routes, dosage forms, administration sites, or the types of excipients and additives, the pharmaceutical composition of the present invention comprises 0.01 to 100% by weight, and in a certain embodiment, 0.01 to 50% by weight of one or more kinds of the crystal or monohydrate of the present invention, as the active ingredient.

The pharmaceutical composition of the present invention can be used in combination with other therapeutic or prophylactic agents which are efficacious for prostate cancer. The combined preparation may be administered simultaneously, or separately and continuously, or at a desired time interval. The preparations to be administered simultaneously may be a mixture, or may be prepared individually.

[Examples]

Hereinafter, the present invention will be described in detail with reference to Examples, but the present invention is not limited thereto.

Example 1

(A01-type crystal of monohydrate of Compound A)

To 250 mg of Compound A produced in conformity to Examples 3 to 9 of Patent Document 1 was added 6 ml of 50% acetonitrile water at room temperature, followed by heating to make a gentle reflux state while stirring, and cooling to room temperature. After cooling, the precipitated solid was collected by filtration and 111 mg (44%) of A01 type crystals of monohydrate of Compound A was obtained in a form of beige colored powder.

The results of element analysis of crystals obtained in Example 1 above are shown in Table 1.

[Table 1]

| | $C_{21}H_{23}ClN_6O.H_2O$ | C (%) | H (%) | N (%) | Cl (%) |
|---|---|---|---|---|---|
| A01-type crystal of monohydrate of Compound A | Calculated value | 58.81 | 5.87 | 19.59 | 16.25 |
| | Measured value | 58.91 | 5.79 | 19.72 | 16.24 |

Example 2

(A01-type crystal of hydrochloride of Compound A)

To 120 mg of Compound A produced in conformity to Examples 3 to 9 of Patent Document 1 were added 1.2 ml of acetonitrile and 0.08 ml of 4M hydrogen chloride/ethyl acetate solution under ice cooling followed by stirring at room temperature overnight. The precipitated solid was collected by filtration and dried under reduced pressure, and 119 mg (91 %) of A01 type hydrochloride crystals of Compound A was obtained in a form of white colored powder.

Example 3

(A01-type crystal of hydrobromide of Compound A)

To 616 mg of Compound A produced in conformity to Examples 3 to 9 of Patent Document 1 were added 6 ml of acetonitrile and 265 mg of 48% hydrobromic acid under ice cooling followed by stirring under ice cooling for one hour. The precipitated solid was collected by filtration and dried under reduced pressure, and 673 mg (91 %) of beige colored powder was obtained.

A mixture of 150 mg of the obtained beige colored powder and 10 ml of 2-propanol were stirred for 10 minutes under heating to reflux, and then stirred for 2 hours at room temperature. The precipitated solid was collected by filtration, and dried under reduced pressure, and then 132 mg (88%) of A01-type crystal of hydrobromide of Compound A was obtained in a form of beige colored powder.

The results of element analysis of crystals obtained in Example 3 above are shown in Table 2.

[Table 2]

| | $C_{21}H_{23}ClN_6O.HBr$ | C (%) | H (%) | N (%) | Cl (%) | Br (%) |
|---|---|---|---|---|---|---|
| A01-type crystal of hydrobromide of Compound A | Calculated value | 51.28 | 4.92 | 17.09 | 7.21 | 16.25 |
| | Measured value | 51.46 | 4.94 | 17.17 | 7.29 | 16.24 |

Example 4

(A01-type crystal of methanesulfonate of Compound A)

To 411 mg of Compound A produced in conformity to Examples 3 to 9 of Patent Document 1 were added 5 ml of acetonitrile and 0.068 ml of methanesulfonic acid followed by stirring at room temperature for one hour. The precipitated solid was collected by filtration and dried under reduced pressure, and 350 mg (69%) of A01-type crystal of methanesulfonate of Compound A was obtained in a form of beige powder.

The results of element analysis of crystals obtained in Example 4 above are shown in Table 3.

[Table 3]

| | $C_{21}H_{23}ClN_6O.CH_4O_3S.\ 0.4H_2O$ | C (%) | H (%) | N (%) | Cl (%) | S (%) |
|---|---|---|---|---|---|---|
| A01-type crystal of methanesulfonate of Compound A | Calculated value | 51.39 | 5.45 | 16.34 | 6.89 | 6.24 |
| | Measured value | 51.24 | 5.22 | 16.38 | 6.86 | 6.46 |

In regard to the crystals obtained in Examples 1 to 4 described above, hygroscopicity, solid stability with respect to the temperature, solid stability with respect to the humidity, and photostability were measured using methods described below.

(Hygroscopicity test)

Measurement of the hygroscopicity of the crystals of the present invention was performed by using VTI SGA-100 under the conditions of temperature: 25°C, measurement range: 5% to 95% relative humidity, and measurement interval: 5% relative humidity.

(Solid stability)

With respect to solid state stability of the crystals of the present invention, physical/chemical stability under temperature and humidity conditions, and chemical stability in a light irradiation state were evaluated. The physical stability was evaluated by the powder X-ray diffraction and the differential scanning calorimetry analysis (DSC) (under the above-described measurement conditions). The chemical stability was evaluated by HPLC (related substance test and optical isomer separating test), and represented by the ratio (%) of generated impurities.

(Solid state stability, temperature)

In an Incubator IC-450A (manufactured by AS ONE Corporation) set as 70°C, a sample was plugged up and stored under the uncontrolled humidity environment for two weeks.

(Solid state stability, temperature/humidity)

A desiccator whose relative humidity was adjusted to approximately 75% by using saturated brine was put into an Incubator IC-450A (manufactured by AS ONE Corporation) set as 70°C, and a sample was unplugged therein and then stored for two weeks.

(Solid stability, irradiation with light)

In a light stability test chamber LTL-200D3J-15, a sample was stored such that integrated illuminance became 1,200,000 lux-hours (temperature: 25°C).

(HPLC measurement condition)
(Related substance test)
Device: LC1100 (manufactured by Agilent Technologies)
Mobile phase: A: 0.01 mol/L perchloric acid buffer solution (pH 2.5), B: MeCN
Flow rate: 0.2 mL/min
Column: Inertsil ODS-3 3 μm (2.1 mm x 100 mm)
Column temperature: 40°C
Gradient condition: Bconc. 20% to 90% (0 to 18 min), 90% (18 to 23 min), 20% (23.1 to 33 min)
Injection volume: 1 μL
Detection wavelength: 254 nm
(Optical isomer separating test)
Device: LC1100 (manufactured by Agilent Technologies)
Mobile phase: mixture of hexane/ethanol (7:3)
Flow rate: 0.5 mL/min
Column: CHIRALPAK AS-H 5 μm (4.6 mm x 250 mm)
Column temperature: 40°C
Injection volume: 10 μL
Detection wavelength: 254 nm

(Solubility)

0.5 mg to 1 mg of a sample was weighed in 10 mL of a centrifuge tube, and respective solutions (see below) were added thereto such that the amount thereof became approximately 150 μg/mL. In order to disperse the sample uniformly, the solution was sonicated in a water bath for a short period of time, followed by shaking at room temperature for 10 minutes using a shaker SA31 (manufactured by Yamato Scientific Co., Ltd.). The solution after being shaken was filtered with a filter, and the concentration of the compound in the filtrate was determined by HPLC. The following solutions were used.

- JP1 (first solution of Japanese Pharmacopoeia Disintegration Test): 2.0 g of sodium chloride was dissolved in 7.0 mL of hydrochloric acid and water and the amount thereof was set to 1000 mL (pH: approximately 1.2).
- JP2 (second solution of Japanese Pharmacopoeia Disintegration Test): 118 mL of 0.2 mol/L sodium hydroxide test solution and water were added to 250 mL of 0.2 mol/L potassium dihydrogen phosphate test solution and the amount thereof was set to 1000 mL (pH: approximately 6.8).
- JP2 + bile acid (JP2 + TC): sodium taurocholic acid was added to the second solution of Japanese Pharmacopoeia Disintegration Test such that the amount thereof became 15 mmol/L.

- FaSSIF (simulated intestinal fluid (fasted state)): 3 mmol/L of sodium taurochloric acid, 0.75 mmol/L of lecithin, 3.9 g of potassium dihydrogen phosphate, and 7.7 g of potassium chloride were added to 1 L of water and the pH thereof was adjusted to approximately 6.5 using sodium hydroxide.
- FeSSIF (simulated intestinal fluid (fed state)): 15 mmol/L of sodium taurochloric acid, 3.75 mmol/L of lecithin, 8.65 g of acetic acid, and 15.2 g of potassium chloride were added to 1 L of water and the pH thereof was adjusted to approximately 5.0 using sodium hydroxide.

(HPLC condition)

Device: LC1100 (manufactured by Agilent Technologies)
Mobile phase: A: 0.01 mol/L aqueous ammonium acetate solution, B: 0.01 mol/L ammonium acetate methanol solution
Flow rate: 0.3 mL/min
Column: Developsil Combi-RP-5.5 μm (2.1 mm x 50 mm)
Column temperature: 40°C
Gradient condition: Bconc. 10% to 50% (0 to 0.5 min), 50% to 100% (0.5 to 5 min), 100% (5 to 7 min), 10% (7.1 to 11.5 min)
Injection volume: 10 μL
Detection wavelength: 254 nm

The solubility and the hygroscopicity of the crystals of the present invention measured by the above-described measurement method are shown in Table 4.

[Table 4]

| | Solubility (μg/ml) JP1/JP2/JP2+TC/FaSSIF/FeSSIF | Hygroscopicity |
|---|---|---|
| A01-type crystal of monohydrate of Compound A | 21.8/1.6/8.8/4.4/17.7 | 0.1% or less |
| A01-type crystal of hydrochloride of Compound A | 9.7/10.1/92.8/39.4/>100 | Approximately 1.4% |
| A01-type crystal of hydrobromide of Compound A | 12.3/19.8/>80.6/41.0/94.9 | 0.1% or less |
| A01-type crystal of methanesulfonate of Compound A | >100/20.6/>100/47.2/>100 | Approximately 5% |

The solid stability (physical) and the solid stability (chemical) of the crystals of the present invention evaluated by the above-described crystal evaluation method are shown in Table 5.

[Table 5]

| | Physical solid stability | Chemical solid stability |
|---|---|---|
| A01-type crystal of monohydrate of Compound A | Stable to the temperature Stable to the temperature/humidity | Temperature 0% Temperature/humidity 0% Light 0.01% |

(continued)

| | Physical solid stability | Chemical solid stability |
|---|---|---|
| A01-type crystal of hydrochloride of Compound A | Stable to the temperature Slightly unstable to the temperature/humidity (volatilization of HCl) | Temperature 0.26% Temperature/humidity 0.02% Light 4.44% |
| A01-type crystal of hydrobromide of Compound A | Stable to the temperature Slightly unstable to the temperature/humidity (volatilization of HBr and polymorphic transition) | Temperature 0.04% Temperature/humidity 0.08% Light 5.3% |
| A01-type crystal of methanesulfonate of Compound A | Slightly unstable to the temperature Unstable to the temperature/humidity (deliquescence) | Temperature 0.13% Temperature/humidity 48.46% Light 2.14% |

Hereinbefore, as described in Examples, the "A01-type crystal of monohydrate", the "A01-type crystal of hydrochloride", "A01-type crystal of hydrobromide", and "A01-type crystal of methanesulfonate" of Compound A, which are the crystals of the present invention, can be produced as certain crystals under particularly controllable conditions, and it is confirmed that the crystals of the present invention are crystals suitable for industrialization in various parameters such as solubility, hygroscopicity, physical solid stability, and chemical solid stability as shown in Tables 4 and 5. In addition, since crystal polymorphism is not confirmed in the "A01-type crystal of monohydrate" of Compound A, the "A01-type crystal of monohydrate" of Compound A is used as an excellent crystal suitable for industrialization, in which a uniform crystal having a certain degree of quality can be obtained with excellent reproducibility and which can be stably supplied as a crystal of a bulk drug used for production of pharmaceuticals.

The AR antagonistic action and the prostate-shrinking action of the crystals of the present invention were confirmed by the following pharmacological test.

(Pharmacological test)

Test example 1: Inhibitory action on transcription activation of human wild type, W741 C mutant and T877A mutant ARs

A vector expressing W741C (mutant AR type exhibiting resistance to bicalutamide which is an AR antagonistic drug) or T877A (mutant AR type exhibiting resistance to flutamide which is an AR antagonistic drug) mutant AR (pSG5-W741C-hAR or pcDNA3.1-T877A-hAR) was transfected into CHO-K1 cells, thereby obtaining human W741C and T877A mutant AR stably expressing cells. Further, human W741C and T877A mutant AR stably expressing cells were obtained by tranfecting luciferase reporter vector into these cells such that luciferase was expressed when AR was activated.

2 x $10^4$ of Wild type (wild type, WT), W741 C, or T877A mutant AR stably expressing CHO-K1 cells were respectively seeded in a 96 well cell culture lumino plate, and incubated at 37°C overnight, and then test compounds having various concentrations were added thereto, together with DHT (final concentration of 0.3 nM). Subsequently, the resultant was further incubated at 37°C overnight, and the amount of luminescence of cells treated with the luciferase assay system was measured using a Luminometer and then set as luciferase activity due to WT, W741C, or T877A mutant AR transcription activation.

According to Example 1, inhibitory action of the crystals of the present invention on the transcription activity was evaluated as luciferase inhibitory activity. $IC_{50}$ value was calculated by Sigmoid-Emax model non-linear regression analysis.

As the test compounds of the above-described test, inhibitory action on the transcription activity of the respective receptors of WT AR (wild type AR), W741C mutant AR, and T877A mutant AR was evaluated using respective crystals of the present invention. For example, $IC_{50}$ value for the AR receptor activity inhibitory action of the A01-type crystal of monohydrate of Compound A is as shown in Table 6.

[Table 6]

| | $IC_{50}$ |
|---|---|
| WTAR | 35 nM |
| W741C mutant AR | 87 nM |
| T877A mutant AR | 39 nM |

Test example 2: prostate-shrinking action with respect to mature male rat

The crystals of the present invention dissolved in 25% propylene glycol/25% Tween 80/50% purified water were continuously and orally administered to 9- to 10-week Wistar male rats twice a day for 15 days or for 36 days. After 6 hours from the final administration, the wet weight of ventral prostate was measured and the prostate shrinking action of the compound of the present invention was examined.

A group to which the crystals of the present invention was administered was set as a test group and a group to which 25% propylene glycol/25% Tween 80/50% purified water was administered was set as a control group, and the prostate-shrinking action of the crystals of the present invention was calculated by the following calculation formula.

$$\text{Reduction ratio (\%)} = 100(B-A)/B$$

A: ventral prostate wet weight of test group
B: ventral prostate wet weight of control group

ED50 value was calculated by linear regression method from the acquired reduction ratio. As a result, the ED50 value of the A01-type crystal of monohydrate of Compound A was 2.4 mg/kg when the crystal was administered twice a day for 15 days and was 0.56 mg/kg when the crystal administered twice a day for 36 days.

Hereinbefore, according to Examples 1 and 2, it was confirmed that the crystals of the present invention were effective as a bulk drug of the AR antagonistic drug and can be used as a drug particularly for treating prostate cancer. As described in Test Example 1, it was confirmed that the crystals of the present invention showed the antagonistic action with respect to wild type AR as well as ARs having resistance to the AR antagonistic drugs bicalutamide and flutamide and can be used as a drug for treating prostate cancer of W741C mutation and T877A mutation in addition to the prostate cancer having wild type AR.

Further, according to the reproducibility evaluation, it was confirmed that the crystals of the present invention have certain properties which are suitable for a bulk drug of pharmaceutical products and can be stably supplied with a certain quality and can be industrialized.

The crystal of (2R,5S)-4-(3-chloro-4-cyanophenyl)-N-(2-cyclopropylpyrimidin-5-yl)-2,5-dimethylpiperazine-1-carboxamide according to the present invention is suitable for industrialization as a bulk drug of pharmaceutical products and is useful as an AR antagonistic drug in which the crystal is used as an active ingredient since the crystal of the present invention has excellent properties in terms of solubility, storage stability, and producing reproducibility, and has the AR antagonistic action, excellent oral activity, and the prostate shrinking action in rats in the above-described test in which the crystal of the present invention was used as a test drug.

## Claims

1. (2R,5S)-4-(3-chloro-4-cyanophenyl)-N-(2-cyclopropylpyrimidin-5-yl)-2,5-dimethylpiperazine-1-carboxamide monohydrate.

2. A crystal of (2R,5S)-4-(3-chloro-4-cyanophenyl)-N-(2-cyclopropylpyrimidin-5-yl)-2,5-dimethylpiperazine-1-carboxamide monohydrate.

3. The crystal according to claim 2, which has peaks at approximately 14.9°, 15.4°, 20.1°, 20.7°, 24.1°, 25.4°, 26.5°, 27.5°, and 29.8° of diffraction angles 2θ (°) in powder X-ray diffraction (tube: Cu).

4. A crystal of (2R,5S)-4-(3-chloro-4-cyanophenyl)-N-(2-cyclopropylpyrimidin-5-yl)-2,5-dimethylpiperazine-1-carboxamide hydrochloride.

5. The crystal according to claim 4, which has an endothermic peak having an extrapolated onset temperature which falls within the range of 160°C to 169°C in the differential scanning calorimetry analysis (DSC analysis).

6. The crystal according to claim 4, which has peaks at approximately 13.2°, 16.2°, 19.5°, 24.7°, 25.1°, 26.1°, and 27.7° of diffraction angles 2θ (°) in powder X-ray diffraction (tube: Cu).

7. The crystal according to claim 4, which has an endothermic peak having an extrapolated onset temperature which falls within the range of 160°C to 169°C in the differential scanning calorimetry analysis (DSC analysis), and peaks

at approximately 13.2°, 16.2°, 19.5°, 24.7°, 25.1°, 26.1°, and 27.7° of diffraction angles 2θ (°) in powder X-ray diffraction (tube: Cu).

**8.** A crystal of (2R,5S)-4-(3-chloro-4-cyanophenyl)-N-(2-cyclopropylpyrimidin-5-yl)-2,5-dimethylpiperazine-1-carboxamide hydrobromide.

**9.** The crystal according to claim 8, which has an endothermic peak having an extrapolated onset temperature which falls within the range of 185°C to 195°C in the differential scanning calorimetry analysis (DSC analysis).

**10.** The crystal according to claim 8, which has peaks at approximately 16.0°, 19.3°, 21.6°, 23.0°, 24.0°, 24.4°, 25.0°, 26.2°, 27.2°, and 30.4° of diffraction angles 20 (°) in powder X-ray diffraction (tube: Cu).

**11.** The crystal according to claim 8, which has an endothermic peak having an extrapolated onset temperature which falls within the range of 185°C to 195°C in the differential scanning calorimetry analysis (DSC analysis), and peaks at approximately 16.0°, 19.3°, 21.6°, 23.0°, 24.0°, 24.4°, 25.0°, 26.2°, 27.2°, and 30.4° of diffraction angles 2θ (°) in powder X-ray diffraction (tube: Cu).

**12.** A crystal of (2R,5S)-4-(3-chloro-4-cyanophenyl)-N-(2-cyclopropylpyrimidin-5-yl)-2,5-dimethylpiperazine-1-carboxamide methanesulfonate.

**13.** The crystal according to claim 12, which has an endothermic peak having an extrapolated onset temperature which falls within the range of 185°C to 195°C in the differential scanning calorimetry analysis (DSC analysis).

**14.** The crystal according to claim 12, which has peaks at approximately 16.6°, 18.3°, 20.3°, 23.9°, 24.4°, 27.1°, and 28.1° of diffraction angles 2θ (°) in powder X-ray diffraction (tube: Cu).

**15.** The crystal according to claim 12, which has an endothermic peak having an extrapolated onset temperature which falls within the range of 185°C to 195°C in the differential scanning calorimetry analysis (DSC analysis), and peaks at approximately 16.6°, 18.3°, 20.3°, 23.9°, 24.4°, 27.1°, and 28.1° of diffraction angles 2θ (°) in powder X-ray diffraction (tube: Cu).

**16.** A pharmaceutical composition, which comprises the crystal according to any one of claims 2 to 15 or (2R,5S)-4-(3-chloro-4-cyanophenyl)-N-(2-cyclopropylpyrimidin-5-yl)-2,5-dimethylpiperazine-1-carboxamide monohydrate according to claim 1 as an active ingredient.

**17.** The pharmaceutical composition according to claim 16, which is an androgen receptor antagonistic drug.

**18.** The crystal according to any one of claims 2 to 15 or (2R,5S)-4-(3-chloro-4-cyanophenyl)-N-(2-cyclopropylpyrimidin-5-yl)-2,5-dimethylpiperazine-1-carboxamide monohydrate according to claim 1 for treating prostate cancer.

**19.** Use of the crystal according to any one of claims 2 to 15 or (2R,5S)-4-(3-chloro-4-cyanophenyl)-N-(2-cyclopropylpyrimidin-5-yl)-2,5-dimethylpiperazine-1-carboxamide monohydrate according to claim 1 for the manufacture of a pharmaceutical composition for treating prostate cancer.

**20.** A method of treating prostate cancer, which comprises administering to a subject an effective amount of the crystal according to any one of claims 2 to 15 or(2R,5S)-4-(3-chloro-4-cyanophenyl)-N-(2-cyclopropylpyrimidin-5-yl)-2,5-dimethylpiperazine-1-carboxamide monohydrate according to claim 1.

Fig. 1

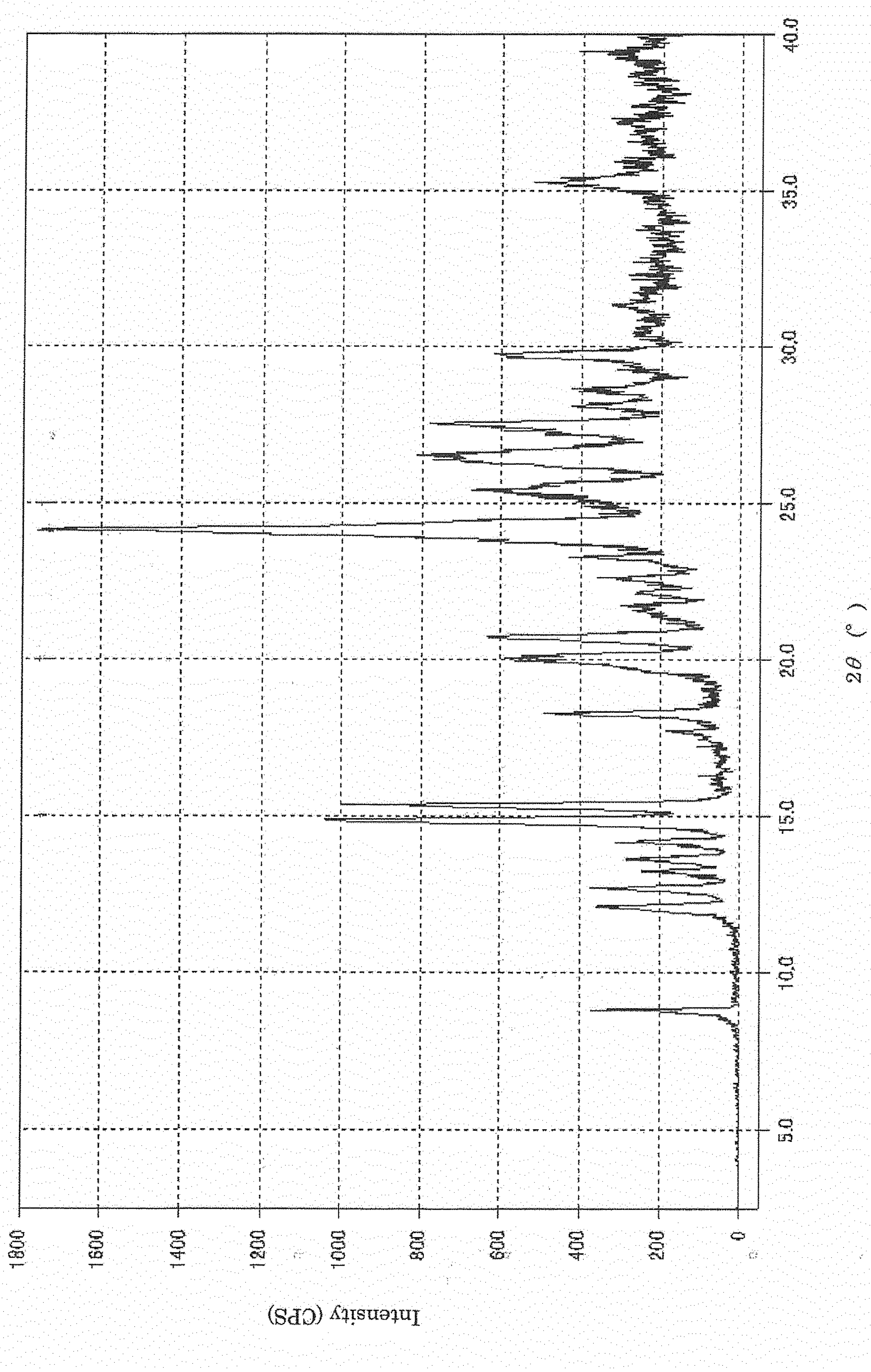
Intensity (CPS)
0
200
400
600
800
1000
1200
1400
1600
1800
2θ (°)
5.0
10.0
15.0
20.0
25.0
30.0
35.0
40.0

Fig. 2

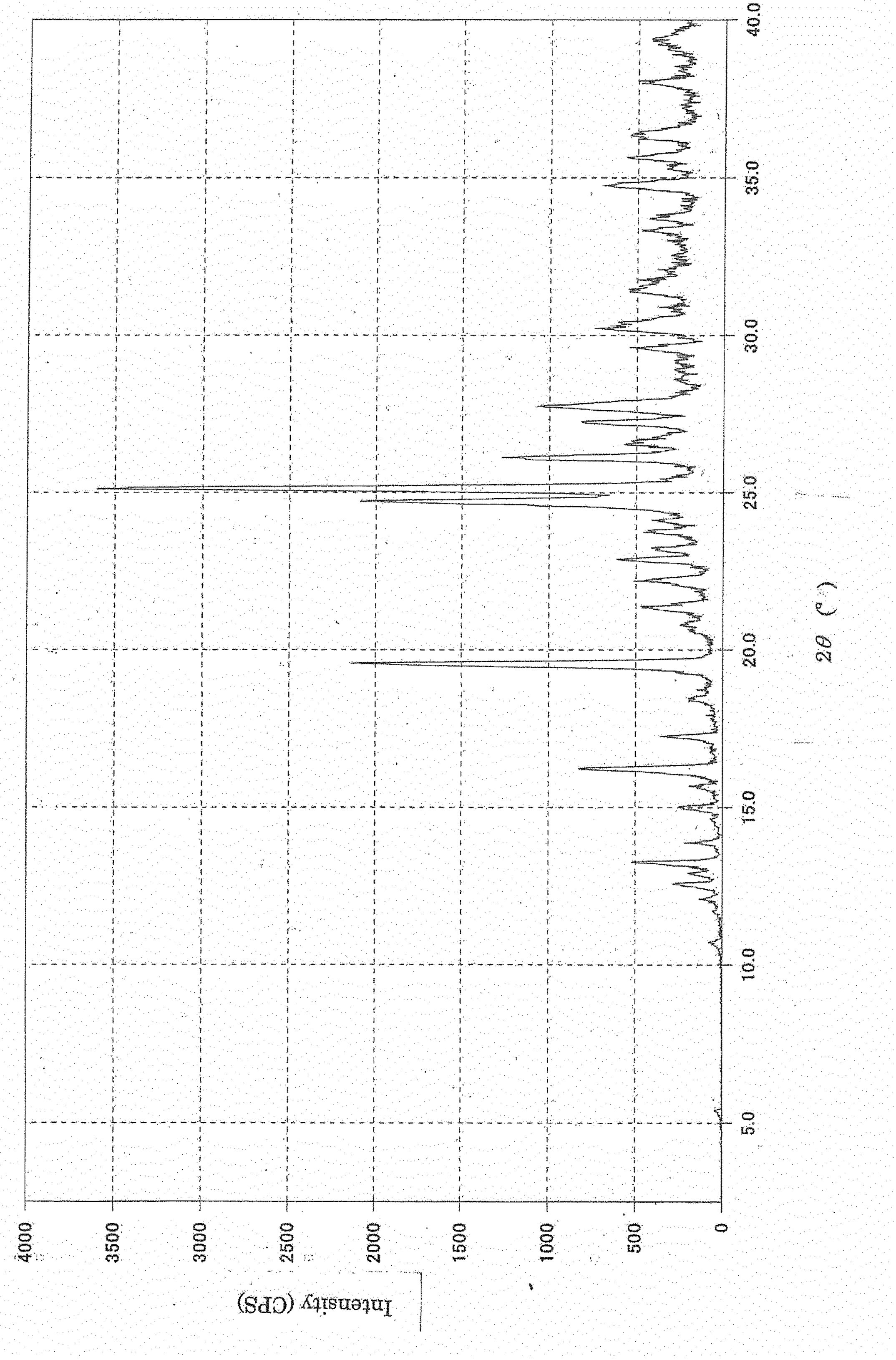
40.0
35.0
30.0
25.0
20.0
15.0
10.0
5.0
2θ (°)
4000
3500
3000
2500
2000
1500
1000
500
0
Intensity (CPS)

Fig. 3

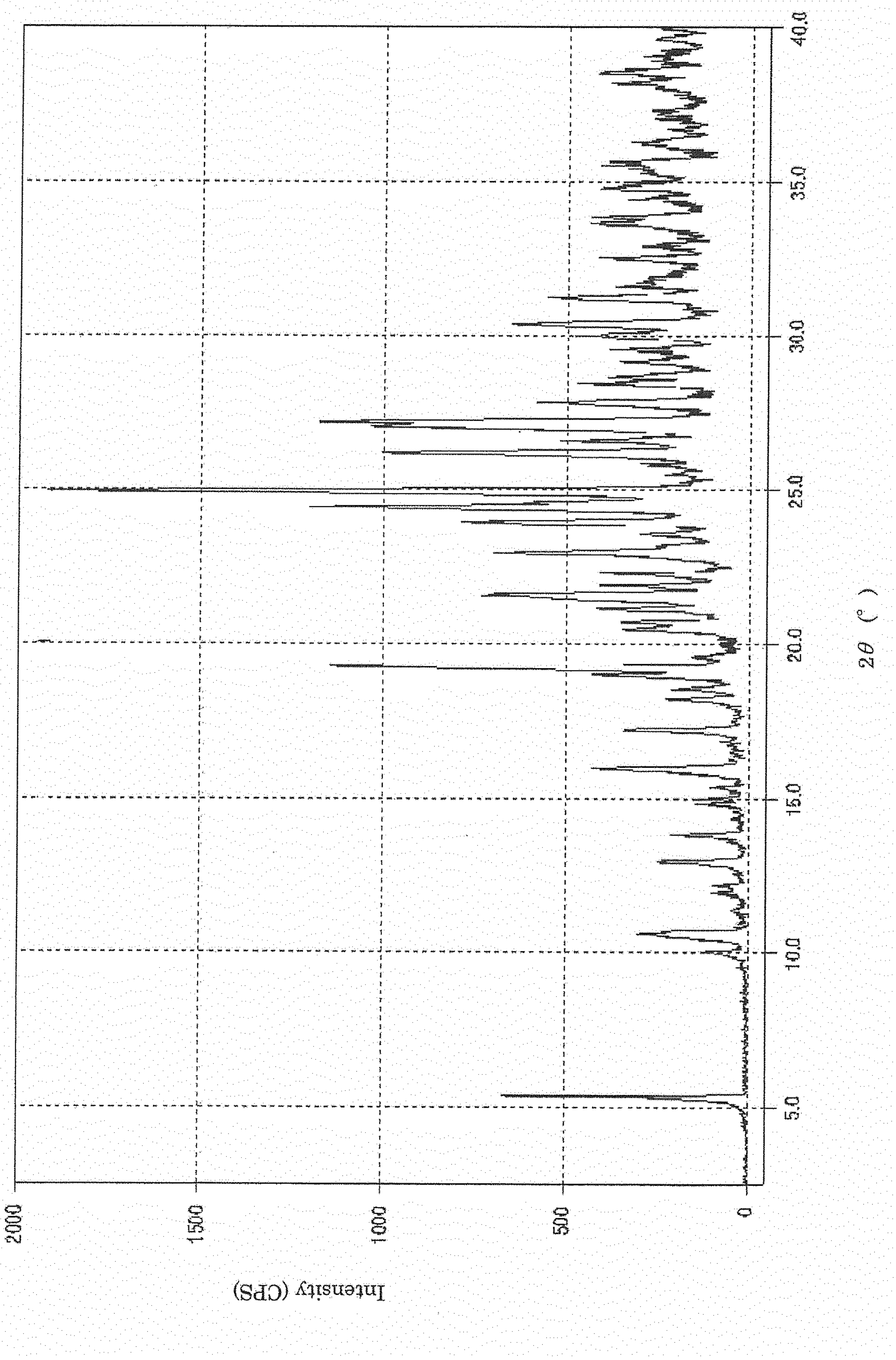
Intensity (CPS)
2θ (°)
0
500
1000
1500
2000
5.0
10.0
15.0
20.0
25.0
30.0
35.0
40.0

Fig. 4

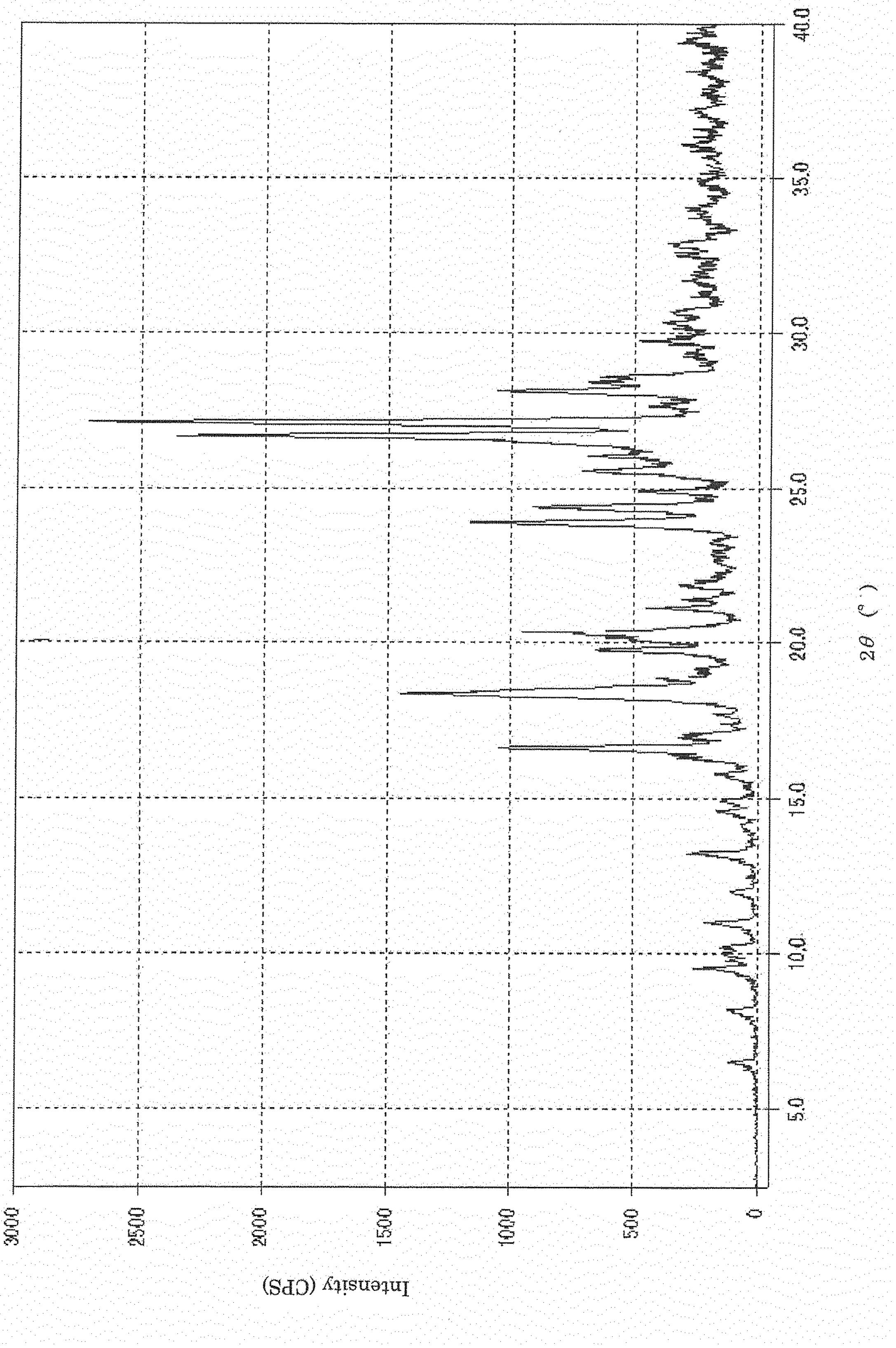
Intensity (CPS)
0
500
1000
1500
2000
2500
3000
2θ (°)
5.0
10.0
15.0
20.0
25.0
30.0
35.0
40.0

Fig. 5

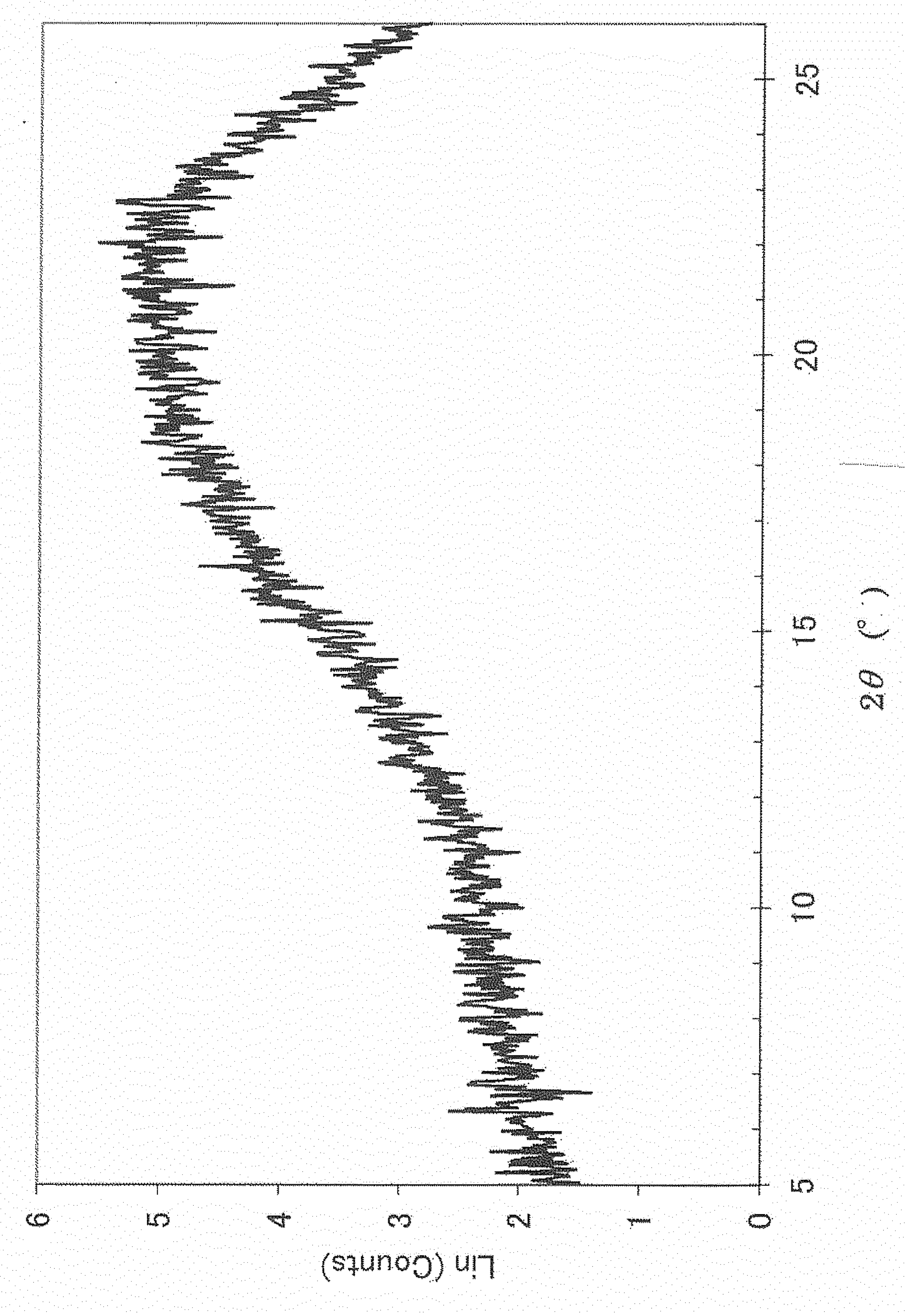

Fig. 6

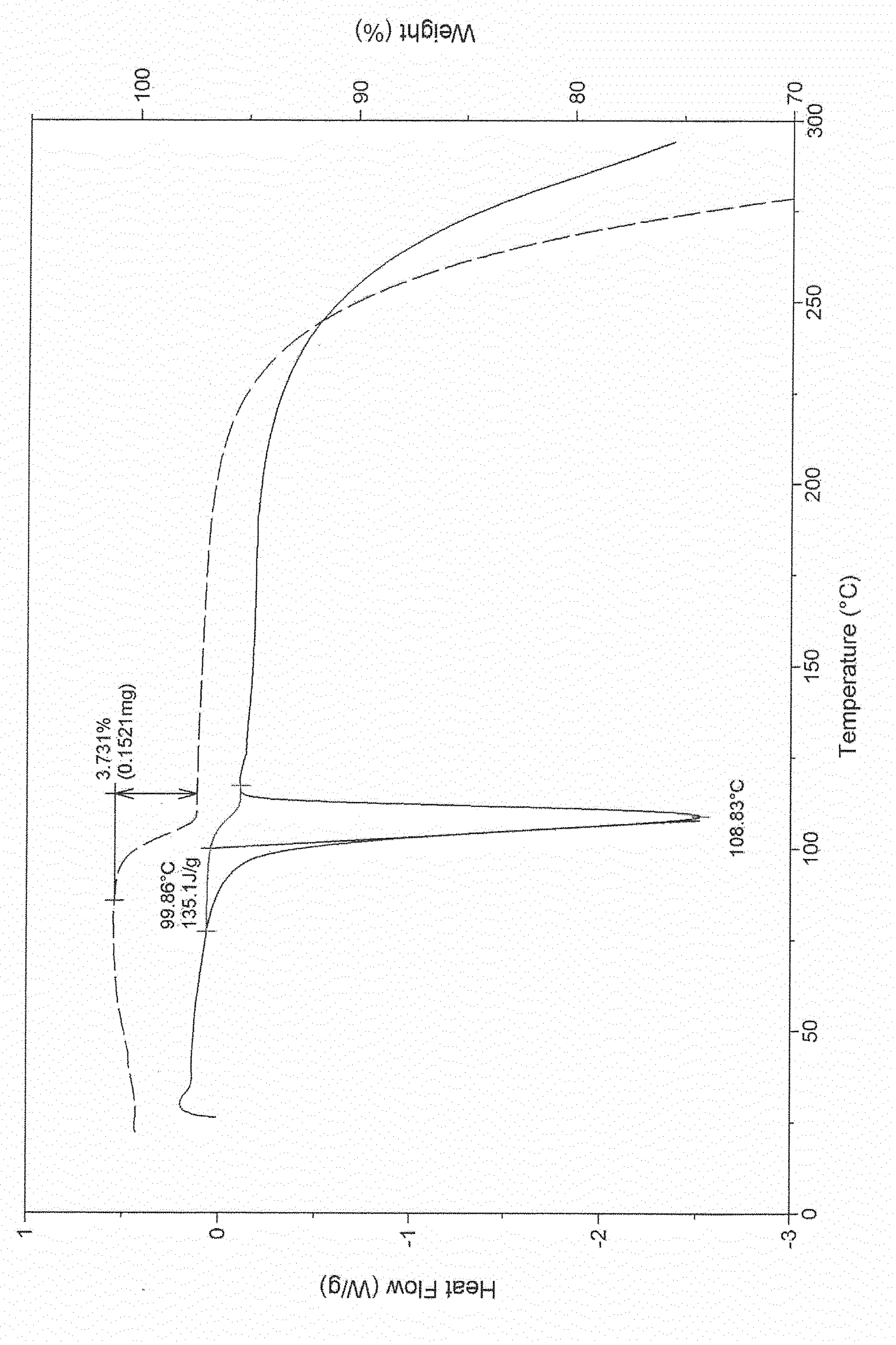
Weight (%)
100
90
80
70
300
250
200
150
100
50
0
Temperature (°C)
3.731%
(0.1521mg)
99.86°C
135.1J/g
108.83°C
1
0
-1
-2
-3
Heat Flow (W/g)

Fig. 7

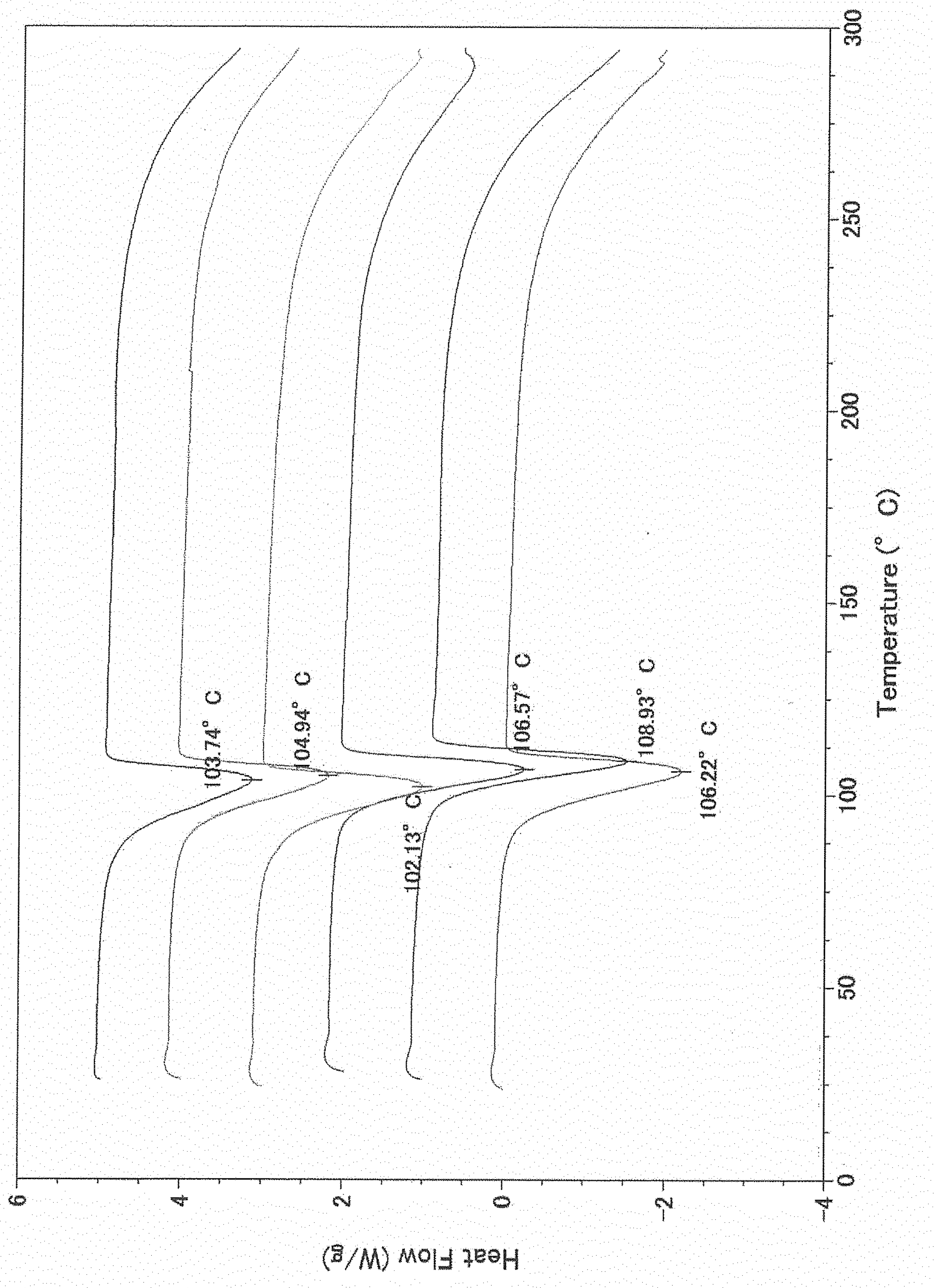
103.74° C
104.94° C
102.13° C
106.57° C
108.93° C
106.22° C
Temperature (° C)
Heat Flow (W/g)
0
50
100
150
200
250
300
6
4
2
0
-2
-4

Fig. 8

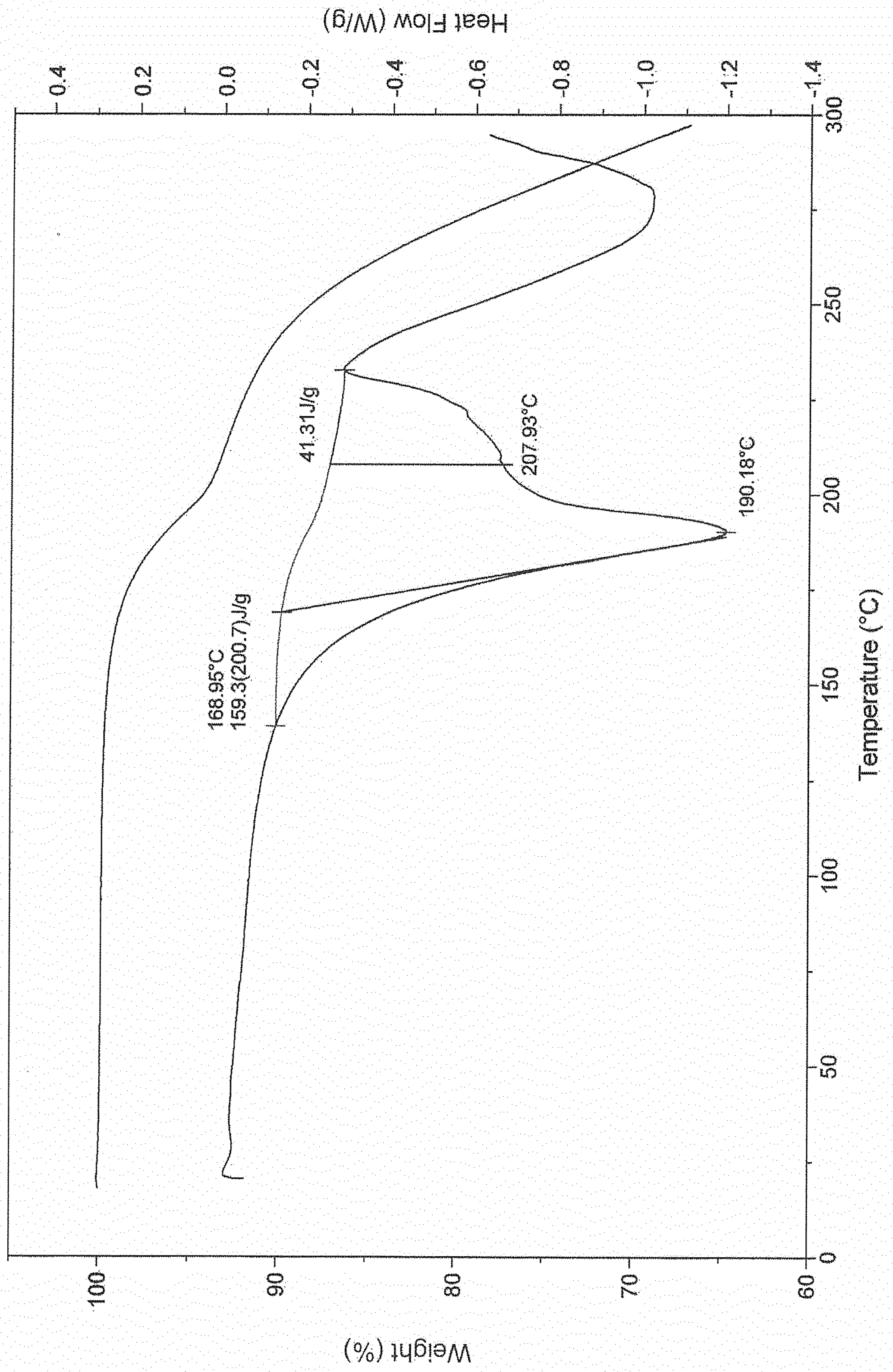
Heat Flow (W/g)
0.4
0.2
0.0
-0.2
-0.4
-0.6
-0.8
-1.0
-1.2
-1.4
Weight (%)
100
90
80
70
60
Temperature (°C)
0
50
100
150
200
250
300
168.95°C
159.3(200.7)J/g
41.31J/g
207.93°C
190.18°C

Fig. 9

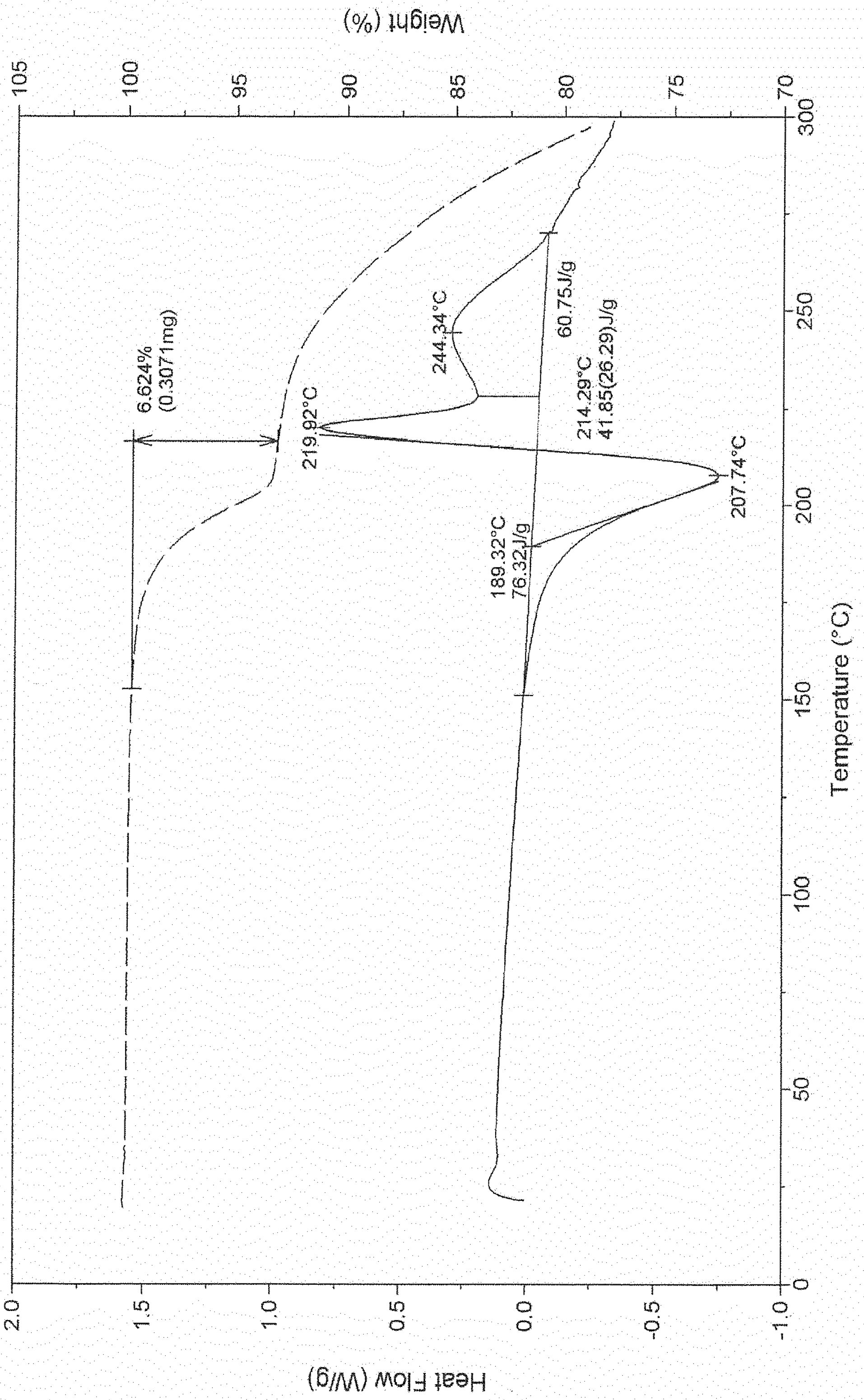
Weight (%)
105
100
95
90
85
80
75
70
6.624%
(0.3071mg)
219.92°C
244.34°C
60.75J/g
214.29°C
41.85(26.29)J/g
207.74°C
189.32°C
76.32J/g
300
250
200
150
100
50
0
Temperature (°C)
2.0
1.5
1.0
0.5
0.0
-0.5
-1.0
Heat Flow (W/g)

Fig. 10

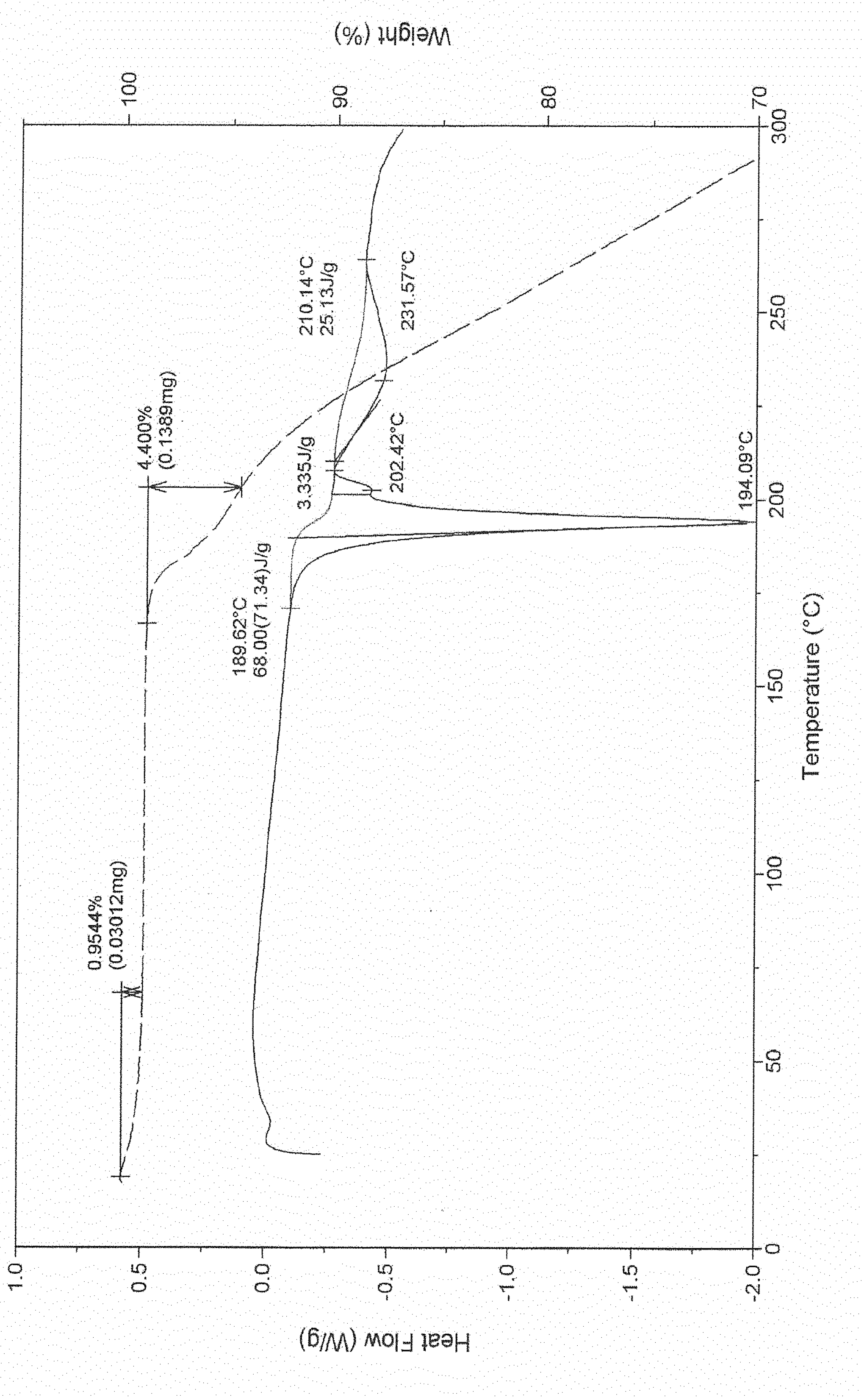
Weight (%)
100
90
80
70
4.400%
(0.1389mg)
0.9544%
(0.03012mg)
210.14°C
25.13J/g
231.57°C
3.335J/g
202.42°C
194.09°C
189.62°C
68.00(71.34)J/g
0
50
100
150
200
250
300
Temperature (°C)
1.0
0.5
0.0
-0.5
-1.0
-1.5
-2.0
Heat Flow (W/g)

Fig. 11

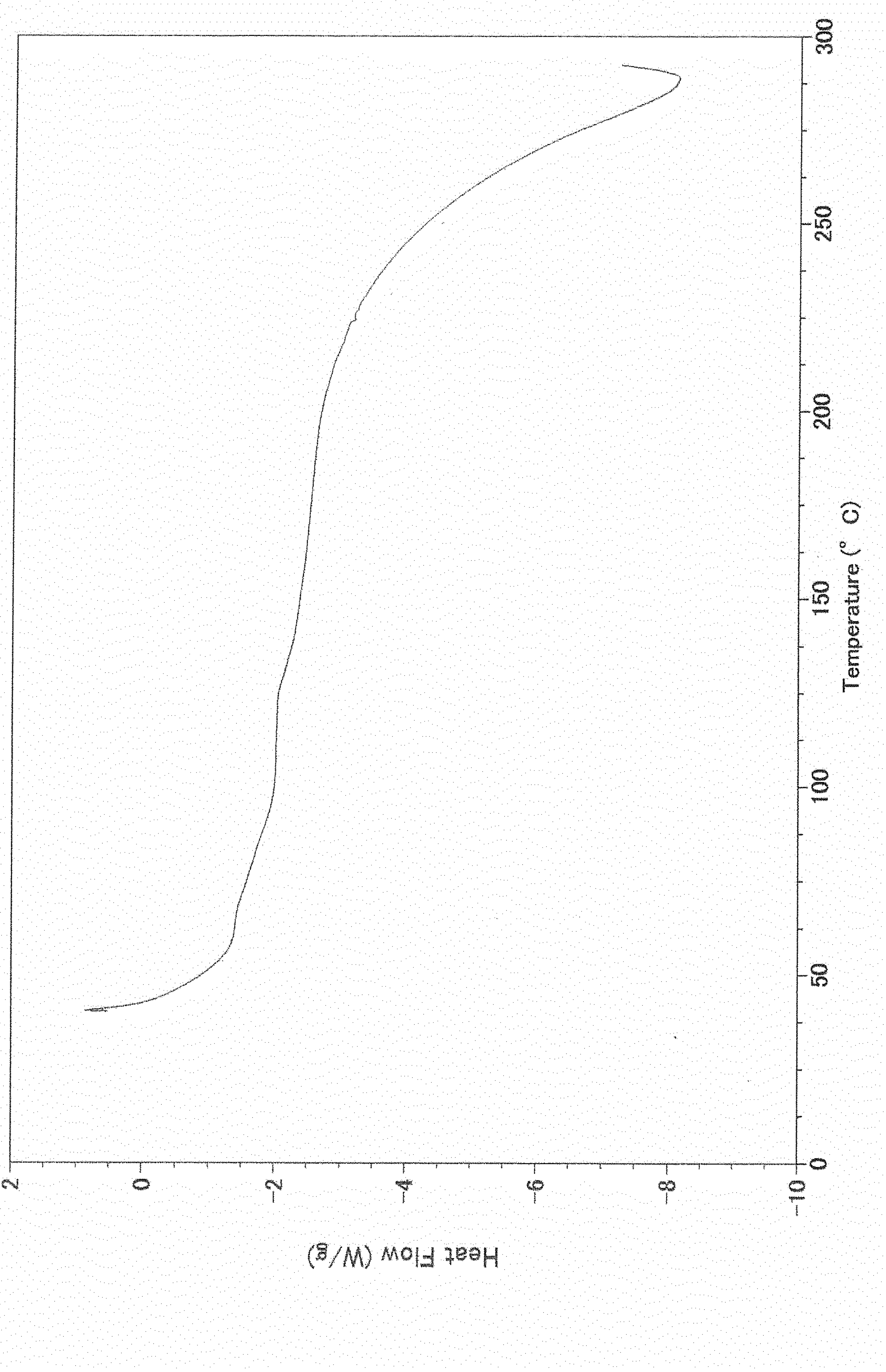

## INTERNATIONAL SEARCH REPORT

International application No. PCT/JP2012/077091

A. CLASSIFICATION OF SUBJECT MATTER

*C07D403/12*(2006.01)i, *A61K31/506*(2006.01)i, *A61P5/28*(2006.01)i, *A61P13/08* (2006.01)i, *A61P35/00*(2006.01)i

According to International Patent Classification (IPC) or to both national classification and IPC

B. FIELDS SEARCHED

Minimum documentation searched (classification system followed by classification symbols)
C07D403/12, A61K31/506, A61P5/28, A61P13/08, A61P35/00

Documentation searched other than minimum documentation to the extent that such documents are included in the fields searched

Electronic data base consulted during the international search (name of data base and, where practicable, search terms used)
CAPLUS/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2004/007471 A1 (ASTELLAS PHARMA INC.), 22 January 2004 (22.01.2004), claims; examples (examples 3-1, 3-7, 3-9); page 8, lines 6 to 20 & JP 4449746 B & US 2005/0261303 A1 & US 2008/0214543 A1 & EP 1557411 A1 | 1-19 |
| Y | Mitsuru HASHIDA, Keiko Toyo Seizai no Sekkei to Hyoka, Yakuji Jihosha, 10 February 1995 (10.02.1995), pages 76 to 79 | 1-19 |
| A | WO 2000/017163 A1 (ASTELLAS PHARMA INC.), 30 March 2000 (30.03.2000), entire text & JP 3390744 B & JP 2003-137873 A & US 2004/0010037 A1 & US 6673799 B1 & EP 1122242 A1 | 1-19 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 06 December, 2012 (06.12.12) | 25 December, 2012 (25.12.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/077091

C (Continuation). DOCUMENTS CONSIDERED TO BE RELEVANT

| Category* | Citation of document, with indication, where appropriate, of the relevant passages | Relevant to claim No. |
|---|---|---|
| A | JP 2001-328938 A (YAMANOUCHI PHARM CO., LTD.), 27 November 2001 (27.11.2001), entire text (Family: none) | 1-19 |
| P,X | WO 2012/053630 A1 (ASTELLAS PHARMA INC.), 26 April 2012 (26.04.2012), entire text (Family: none) | 1-19 |

**INTERNATIONAL SEARCH REPORT**

International application No.
PCT/JP2012/077091

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 20
because they relate to subject matter not required to be searched by this Authority, namely:
Claim 20 pertains to method for treatment of the human body by therapy and thus relates to a subject matter on which this International Searching Authority is not required to carry out a search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004007471 A
- WO 2012053630 A